(19)

**Europäisches Patentamt**
**European Patent Office**
**Office européen des brevets**

(11) **EP 3 861 980 A1**

(12) **EUROPEAN PATENT APPLICATION**

(43) Date of publication:
**11.08.2021 Bulletin 2021/32**

(51) Int Cl.:
*A61K 9/00* (2006.01)     *A61K 35/14* (2015.01)

(21) Application number: **20155897.0**

(22) Date of filing: **06.02.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Ghashghaeinia, Mehrdad**
**70565 Stuttgart (DE)**

(72) Inventor: **Ghashghaeinia, Mehrdad**
**70565 Stuttgart (DE)**

(74) Representative: **Witte, Weller & Partner**
**Patentanwälte mbB**
**Postfach 10 54 62**
**70047 Stuttgart (DE)**

(54) **ACTIVE INGREDIENT OF AN ERYTHROCYTES-CONTAINING COMPOSITION**

(57)     The present invention relates to an active ingredient of an erythrocytes-containing composition, an erythrocytes-containing composition comprising said active ingredient, and a method of treatment of a human or animal being suffering from and/or threatened by blood loss or a blood-formation disorder.

**EP 3 861 980 A1**

**Description**

The present invention relates to an active ingredient of an erythrocytes-containing composition, an erythrocytes-containing composition comprising said active ingredient, and to a method of treatment of a human or animal being suffering from and/or threatened by blood loss or a blood-formation disorder.

FIELD OF THE INVENTION

The invention relates to the field of molecular medicine, more particular the field of molecular blood and transfusion medicine.

BACKGROUND OF THE INVENTION

During blood conservation, leucocyte- and platelets-depleted erythrocyte concentrates are currently stored at 4°C in erythrocyte-specific conservation solutions, the so-called stabilizing additive solutions. Generally, they contain purine-nucleotides like adenine, guanosine or inosine, dextrose (glucose), phosphate including mannitol or sorbitol in sodium chloride solution. The synthesis of ATP and 2,3-BPG is improved during storage by purine-nucleotides, which in turn prolongs the viability of the erythrocytes. The phosphates function as buffer and prevent a lowering of the pH value by lactate. Lactate is produced during the anaerobe glycolysis in erythrocytes. The sugar substitutes mannitol or sorbitol as naturally-occurring polyol isomers regulate the extra- and intracellular osmotic gradients.

It has been shown that the antioxidant N-Acetyl-L-Cysteine (NAC) *in vivo* slows down senescence as well as senescence-dependent cell death of erythrocytes, the so-called eryptosis; see Ghashghaeinia M. et al. 2012; Br. J. Haematol. 157: 606-614, "The impact of erythrocyte age on eryptosis".

It is also known that NAC is immediately taken up by all eukaryotic cell types, among them also red blood cells and is, in combination with the amino acids glycine and glutamate, instantly converted into the vital antioxidant molecule GSH which is the reduced form of glutathione. A continual decrease of GSH or its depletion curbs the functionality of erythrocytes. This happens when oxidative stress and thus the generation of "reactive oxygen species" (ROS) exceeds the redox capacity of the cells. Under these conditions numerous enzymes and structural proteins are deactivated by oxidation, finally causing eryptosis. These processes can be partly reversed by an exogenous NAC addition and its associated GSH production/regeneration.

The members of the transcription factors NFkappaB (NFκB family) are additionally very important players, positively influencing the survival of human erythrocytes. Young human erythrocytes possess large quantities of NFκB whereas the aged human erythrocytes lose large quantities of their NFκB with advancing age, and thus exhibit a higher eryptosis rate; see Ghashghaeinia M. et al. 2013; Cell. Physiol. Biochem. 32: 801-813, "Age sensitivity of NFκB abundance and programmed cell death in erythrocytes induced by NFκB inhibitors".

The signal molecule nitric oxide (NO) is an additional factor positively regulating the survival of human erythrocytes, whereas calcium ($Ca^{++}$) inhibits the pro-survival effect of NO; see Ghashghaeinia M. et al. 2017; Cell. Physiol. Biochem. 42: 1985-1998, "Trifluoperazine-Induced Suicidal Erythrocyte Death and S-Nitrosylation Inhibition, Reversed by the Nitric Oxide Donor Sodium Nitroprusside".

As mentioned above, blood conserves or blood bags are currently supplemented by stabilizers such as acid citrate dextrose with adenine (ACD-A), citrate phosphate derivative (CPD) or citrate phosphate derivative with adenine (CPD-A) to increase their shelf lives. Citrate suppresses blood clotting, phosphate serves to stabilize the pH value, glucose acts as a source of energy, adenine promotes the formation of adenosine triphosphate (ATP) in the red blood cells. The most commonly used stabilizer is the CPD-A solution. It contains 3 g of citric acid, 26.3 g of sodium citrate, 2.2 g of sodium dihydrogen phosphate, 31.9 g of glucose monohydrate and 0.275 g of adenine, which are made up to 1 liter with 1000 ml of distilled water. The pH of this stabilizer solution is between 5.6 and 5.8; if 63-70 ml of it are mixed with 450 ml of blood, the resulting pH value of the preserve is 7.1-7.2. Such an erythrocyte preparation can be usually preserved for 35 to 42 days at a storage temperature of 2-6°C.

A storage of up to 6 weeks and thus availability of blood bags or erythrocyte concentrates is absolutely indispensable for the saving of human lives. However, due to glucose metabolism and long storage periods, the storage is associated by the production of plenty of metabolites, as for instance lactate, toxic methylglyoxal, lipid peroxide, i.e. the so-called reactive oxygen species (ROS), and ROS-related GSH decrease. The metabolic-, senescence- and storage-dependent increase in stress factors in blood bags continuously leads to eryptosis and the intensification of hemolytic processes.

Thus, over time the quality, function and vitality of erythrocytes in blood bags lessens and the quantity of human erythrocytes decreases. As a consequence, the immune system of the respective blood recipient detects a significant part of the blood of the donor as biologically and functionally unsuitable. The harmed erythrocytes must therefore be eliminated by the macrophages of the blood recipient. A certain quantity of the harmed erythrocytes also becomes hemolytic. The released hemoglobin molecules of the blood donor constitute a massive immunological risk to the blood recipient and thus to his/her health.

Therefore, there is a need in the art of compounds which improve the viability and functionality of erythrocytes in erythrocytes-containing solutions, thereby allowing a longer storage of said compositions in transfusion medicine applications.

It is, therefore, an object underlying the invention to provide an active ingredient for an erythrocytes-containing composition capable of avoiding or reducing the above-described disadvantages associated with the current erythrocytes-containing compositions. In particular such an active ingredient is to be provided resulting in a composition which is characterized by erythrocytes displaying a high degree of viability and/or functional efficiency resulting in a reasonable or extended shelf life of said composition.

The present invention satisfies these and other needs.

SUMMARY OF THE INVENTION

The present invention provides the use of chelerythrine or a derivative thereof *in vitro* as an active ingredient of an erythrocytes-containing composition.

The inventor has realized that chelerythrine or a derivative thereof can be used *in vitro* as an active ingredient. In particular, in an experimental set-up where eryptosis and cell shrinkage of erythrocytes was induced by the chemical costunolide, thus simulating phenomena appearing in long stored blood conserves, it was demonstrated by the inventor that chelerythrine can inhibit or even reverse such phenomena. These findings were surprising because in the art chelerythrine is described in a completely different context.

For example, the WO 99/60849 proposes, as one out of a very high number of other components, chelerythrine to be used as an ingredient of a composition configured to the cryopreservation of erythrocytes. Chelerythrine is said of having the function of manipulating phosphorylation events.

Further, the US 5,137,912 discloses that chelerythrine may have an inhibitory effect on the platelet aggregation and could be used to prevent the formation of a thrombus. Chelerythrine is therefore suggested in this document as an anti-blood coagulation agent.

In contrast, the inventor has realized that chelerythrine or a derivative thereof have positive effects on the viability of erythrocytes. Erythrocytes, however, are an important component of the blood coagulation system. A person skilled in the art would associate a positive effect on a component of the blood coagulation system with an improvement in coagulation. However, as the US 5,137,912 indicates a negative effect of chelerythrine on the blood coagulation system the state of the art rather guides away from the invention.

Chelerythrine is further recommended in the art as an antimicrobial and anti-inflammatory agent against oral infections and is described as an inhibitor of rat liver aminotransferase, a potent cytotoxic agent and decoupler of oxidative phosphorylation.

Chelerythrine is also described in the art as a potent, selective and cell-permeable inhibitor of the protein kinase C alpha (PKC-$\alpha$); Herbert J.M. et al. 1990;

Biochem Biophys Res Commun. 172: 993-999, "Chelerythrine is a potent and specific inhibitor of protein kinase C", and Hu B. et al. 2017; Kidney Blood Press Res. 42: 379-388, "Chelerythrine Attenuates Renal Ischemia/Reperfusion-induced Myocardial Injury by Activating CSE/H2S via PKC/NF-κB Pathway in Diabetic Rats".

The findings of the inventor could therefore not be expected.

Chelerythrine, also referred to as 1,2-Dimethoxy-12-methyl[1,3]benzodioxolo[5,6-c]phenanthridinium (CAS number 34316-15-9 or 3895-92-9 (chloride)), with a molecular weight of 383.82 grams per mol, is a chemical compound belonging to the group of quaternary benzophenanthridines. Chelerythrine naturally occurs in the celandine *Chelidonium majus.* It is the most effective alkaloid of the plant with a content of up to 2%. Chelerythrine is also found in other plants such as the California poppy *Eschscholtzia californica* and various species of horn poppy.

A "derivative" of chelerythrine, according to the invention, is a compound which is derived from chelerythrine and still comprises at least 50% of the anti-PKC-$\alpha$-inhibitory activity of chelerythrine. Preferably, in a derivative of chelerythrine one or more of the methyl groups of chelerythrine may be replaced by one or more lower alkyl group(s). One or more of the hydrogen residues may be replaced by one or more halogen group(s). Lower alkyl, according to the invention, includes C1-C5 groups and may be linear or branched. Halogen includes fluorine, chlorine, bromine, and iodine. The molecular weight of the sum of all methyl groups and hydrogen residues which have been replaced is lower than 200 grams per mole.

According to the invention an "active ingredient" refers to a compound that exerts a specific effect in the composition, in particular an inhibitory activity on the erythrocytes' PKC-$\alpha$, as contrasted by non-specific effects, such as e.g. buffering effects.

It is to be understood that the erythrocytes-containing solution, according to the invention, may contain, in addition to chelerythrine and/or its derivatives, above-referenced stabilizers. The erythrocytes may also be dispersed in a standardized erythrocyte-specific preservation solution or "stabilizing additive solution", respectively.

It is also to be understood that the erythrocytes-containing solution according to the invention may contain chelerythrine and in addition one or more of its derivatives.

The inventor found out that, in contrast to what might be thought when referring to US 5,137,912, chelerythrine and derivatives thereof do not negatively affect the physiological activity of erythrocytes. To the contrary, they support the vitality and functional efficiency of erythrocytes.

In a further embodiment of the invention said erythrocytes-containing composition is a blood conserve.

The inventor has realized that his invention can be used to improve the quality and storage stability of blood conserves due to the positive effect of chelerythrine and derivatives thereof on the vitality and functional efficiency of erythrocytes.

According to the invention a "blood conserve" includes a conventional erythrocytes-containing blood bag, especially packed red blood cells, and erythrocytes concentrates. This embodiment transfers the scientific findings of the inventor into a medical product in an advantageous manner.

In an embodiment of the invention the erythrocytes-containing composition and/or blood conserve is configured for a non-cryopreserved storage.

The inventor has realized that, other than what is described in prior art document WO 99/60849, chelerythrine and derivatives thereof are not only suitable as a component of a cryopreservation composition intended for a freezing of erythrocytes at - 80°C. It is beneficial as an active ingredient of an erythrocytes-containing composition or a blood conserve configured and/or intended for a storage in non-frozen or non-cryopreserved state, e.g. at temperatures of approx. 4-6°C.

In another embodiment of the invention the chelerythrine or derivative thereof supports the viability and/or functional efficiency of said erythrocytes.

This measure has the advantage that the chelerythrine or derivatives thereof positively influence the biological activity of the erythrocytes and create an environment allowing a storage over longer times without running the risk of generating stress factors which may lead to eryptosis or hemolytic processes.

For this reason, in another embodiment of the invention said chelerythrine or derivatives thereof inhibit eryptosis and/or hemolysis.

In yet another embodiment of the invention the chelerythrine or derivative thereof increases the shelf-life of said composition and/or blood conserve.

With this measure the inventor contributes to the solution of a serious problem of transfusion medicine which so far has not been satisfactorily dealt with.

In another embodiment of the invention the chelerythrine or derivative thereof is contained in the composition and or blood conserve in a concentration of approx. $\geq 0.5\ \mu$M, preferably of approx. $\geq 1\ \mu$M, further preferably of approx. $\geq 5\ \mu$M, and highly preferably of approx. $\geq 10\ \mu$M.

The inventor has realized that within the indicated concentration ranges a positive effect on the erythrocytes can be seen while at the same time side effects are minimized or even excluded.

Another subject-matter of the invention relates to an erythrocytes-containing composition comprising chelerythrine or a derivative thereof as an active ingredient.

The features, advantages and embodiments disclosed for the use according to the invention apply *mutatis mutandis* to the erythrocytes-containing composition.

In an embodiment of the invention the erythrocytes-containing composition is a blood conserve which is preferably configured for a non-cryopreserved storage.

Another subject-matter of the invention relates to an erythrocytes-containing composition as described further above for use in the treatment and/or prophylaxis of blood loss, preferably acute blood loss, in a human and/or animal being.

The inventor has realized for the very first time that an erythrocytes-containing composition, such as a blood conserve of blood bag, comprising chelerythrine or derivatives thereof as an active ingredient, can be used instead of or in addition to erythrocytes-containing composition currently used in the art for counter-acting or compensating blood loss or a blood-formation disorder. Acute loss of blood, eventually associated with hemorrhagic shock, might occur in cases of serious injuries and excessive bleeding and may result in anemia. Here, the invention can effectively remedy the situation and contribute to improving the condition of the patient.

In another embodiment of the invention the erythrocytes-containing composition is configured for a use in the treatment and/or prophylaxis of a blood-formation disorder.

This measure has the advantage that the invention is adapted to the treatment or attenuation of the symptoms of serious diseases which so far cannot be addressed in the art in a satisfactory manner.

Another subject-matter of the present invention relates to a method of treatment of a human or animal being suffering from and/or threatened by blood loss, preferably acute blood loss, or a blood-formation disorder, comprising the administration of the erythrocytes-containing composition according to the invention.

The features, advantages and embodiments disclosed for the use according to the invention apply *mutatis mutandis* to the method of treatment.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner in the context of any embodiment of the invention.

The invention is now described and explained in further detail by referring to the following non-limiting examples and figures.

Fig. 1: Sketch illustrating problems arising from prolonged blood storage.

Fig. 2: A-B and D-E: Costunolide-induced eryptosis and cell shrinkage in mature human erythrocytes. C: Costunolide induced eryptosis is not associated with hemolysis. Number of independent experiments: n=3.

Fig. 3: Pre-incubation of human erythrocytes with chelerythrine results in inhibition of costunolide-induced eryptosis and cell shrinkage. Incubation time for chelerythrine (26h) and costunolide (24h). Number of independent experiments: n=3.

Fig. 4: Inhibition of glucose depletion-induced eryptosis and cell shrinkage by treatment of human erythrocytes with chelerythrine. Incubation time: 24 h. Number of independent experiments: n=2.

Fig. 5: Complete depletion of both GSH and GSSG after 24 h treatments of human erythrocytes with costunolide. Number of independent experiments: n=3.

Fig. 6: Complete inactivation of glucose-6-phosphate dehydrogenase (G6PDH) activity after 24 h treatment of human erythrocytes with costunolide. Number of independent experiments: n=3.

Fig. 7: Chelerythrine does not affect costunolide-induced GSH- and GSSG-depletion. In order to make the extremely low GSSG values visible, they were indicated as pmol instead of nmol. The incubation time for chelerythrine-treated erythrocytes lasted 26 h and 24h for costunolide-treated erythrocytes. Number of independent tests: n=5.

Fig. 8: Chelerythrine as a potent and selective inhibitor of the protein kinase C alpha (PKC-$\alpha$), inhibits costunolide-induced eryptosis.

EXAMPLES

1. Material and methods

*Study design*

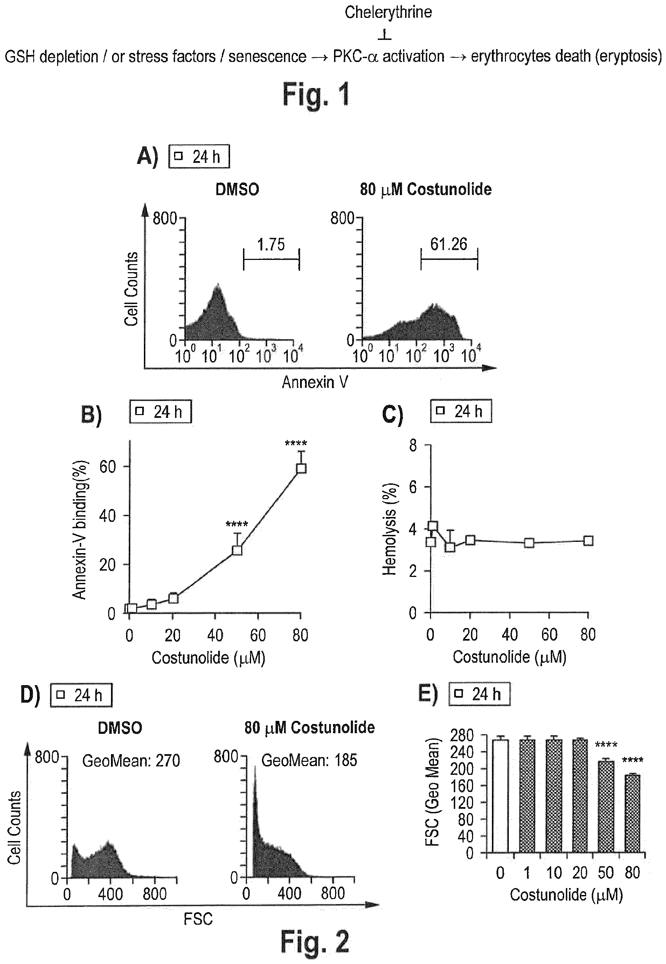

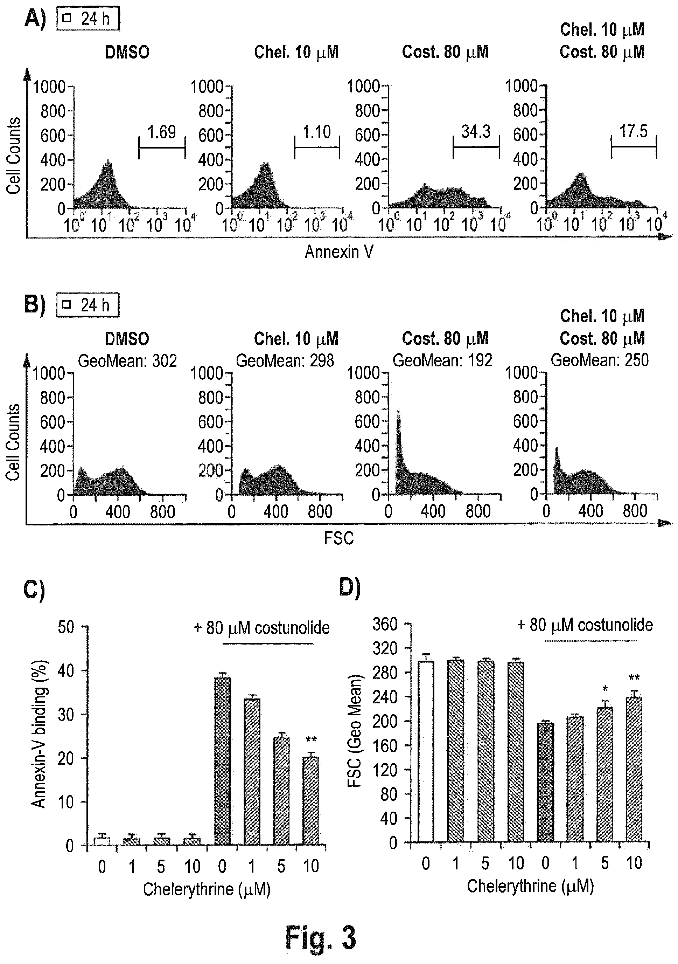

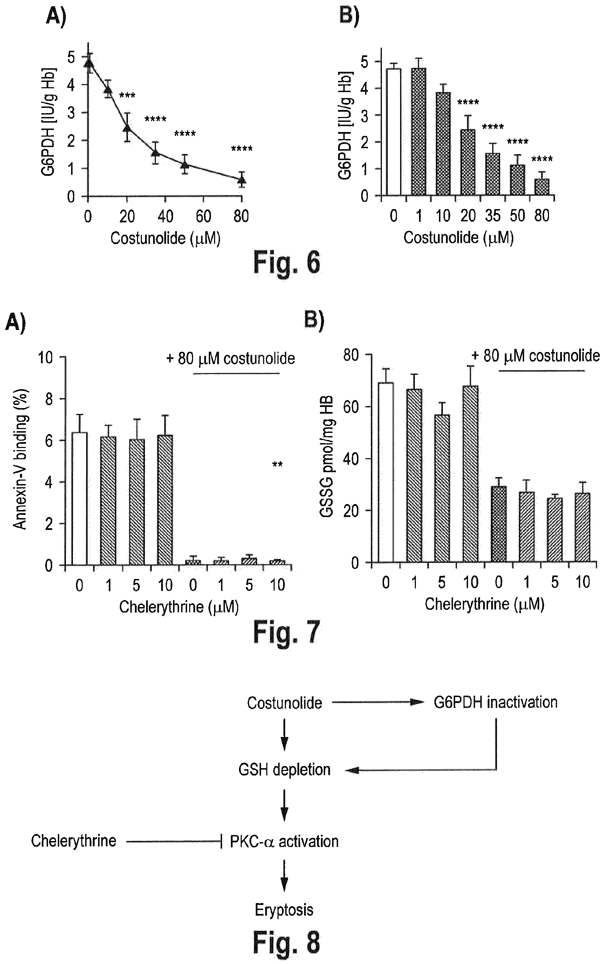

Single treatment: Erythrocytes were treated with costunolide or chelerythrine for 24 hours (see Figures 2, 4, 5 and 6). Double treatments: Erythrocytes were first pre-treated with chelerythrine for two hours, followed by treatment with costunolide for further 24 hours (see Figures 3 and 7). Thus, the incubation time for chelerythrine-treated erythrocytes lasted 26 h and 24h for costunolide-treated erythrocytes. In single (0.2% v/v DMSO) and double treatments (0.4% v/v DMSO), DMSO-treated cells served as negative controls.

*Erythrocytes*

Highly purified erythrocyte suspensions from healthy volunteers with white blood cell (WBC) or thrombocyte contaminations below 0.1% (Lang P.A. et al. 2005; J. Cell. Sci. 118: 1233-1243 "Stimulation of erythrocyte ceramide formation by platelet-activating factor") were provided by the blood bank of the University of Tübingen. Aliquots of the individual erythrocyte concentrates were either used directly at 0.6% hematocrit (Hct) or stored at 4°C for up to one week. The study was approved by the ethics committee of the University of Tübingen, the study was performed in agreement with the declaration of Helsinki, and volunteers gave written consent (184/2003 V).

*Solutions*

Experiments analyzing annexing V binding and cell volume (0.6% Hct) were carried out in Ringer solution. Staining of erythrocytes with annexin-V-FLUOS was performed in annexin binding buffer. Ringer solution was composed of (in mM): 125 NaCl, 5 KCl, 1.2 $MgSO_4$, 32 N-2-hydroxyethyl-piperazine-N'-ethanesulfonic acid (HEPES)/NaOH (pH 7.4), 5 glucose, and 1 $CaCl_2$. Annexin-binding buffer contained (in mM): 125 NaCl, 10 HEPES/NaOH (pH 7.4), and 5 $CaCl_2$.

*Chemicals*

1 mg chelerythrine (chloride) was dissolved in 520 $\mu$l and 5 mg costunolide in 538 $\mu$l DMSO to achieve 5 mM and 40 mM stock solutions, respectively. These stocks were subsequently aliquoted and stored at -20°C for up to one month. Annexin-V-FLUOS was also aliquoted and stored at -20°C for several months. Chelerythrine, costunolide, DMSO, annexin-V-FLUOS, N-Ethylmaleimide (NEM) and EGTA were purchased from Sigma (Taufkirchen, Germany).

*Annexin-V-FLUOS working concentration*

On the day of the measurements the required annexin-V-FLUOS was diluted in annexin binding buffer in a ratio of 1:33. From this batch 48 $\mu$l were taken to stain each sample with $3\times10^6$ erythrocytes (for more details see flow cytometry).

*Flow cytometry*

At the end of the incubation period, 0.1 ml erythrocytes ($3\times10^6$) were added in 500 $\mu$l annexin wash buffer, mixed thoroughly and pelleted by centrifuging. Erythrocytes pellets were gently vortexed to obtain a homogeneous cell suspension. To detect the exposure of phosphatidylserine (PS) on the outer leaflet of the plasma membrane (a measure of the intensity of eryptosis), erythrocytes were stained with 48 $\mu$l of diluted annexin-V-FLUOS and carefully vortexed. After an incubation period of 20 minutes in the dark at room temperature, 200 $\mu$l annexin binding buffer was added to each sample, thoroughly vortexed to obtain single cell suspensions, and analyzed by flow cytometry on a FACS Calibur (Becton Dickinson, Heidelberg, Germany). In the FL1-channel, binding of annexin-V-FLUOS (eryptosis) was measured. Erythrocyte volume was determined by forward scatter (FSC). To this end, corresponding erythrocytes suspensions were immediately analysed by flow cytometry.

*Hemolysis measurement*

After 24 hours treatment of erythrocytes (0.6% Hct) with costunolide and/or chelerythrine, hemolysis was determined. 600 $\mu$l cell suspension form each condition containing 3.6 x 106 erythrocytes centrifuged for 8 min at 420 x g, 4 °C, and the supernatants were harvested. As a measure of hemolysis, the hemoglobin (Hb) concentration of the supernatants was determined photometrically at 405 nm. The absorption of the supernatant of DMSO-treated erythrocytes lysed in 600 $\mu$l distilled water was defined as 100%. From this the standard curve was made by serial dilution.

*Intracellular GSH and GSSG analysis*

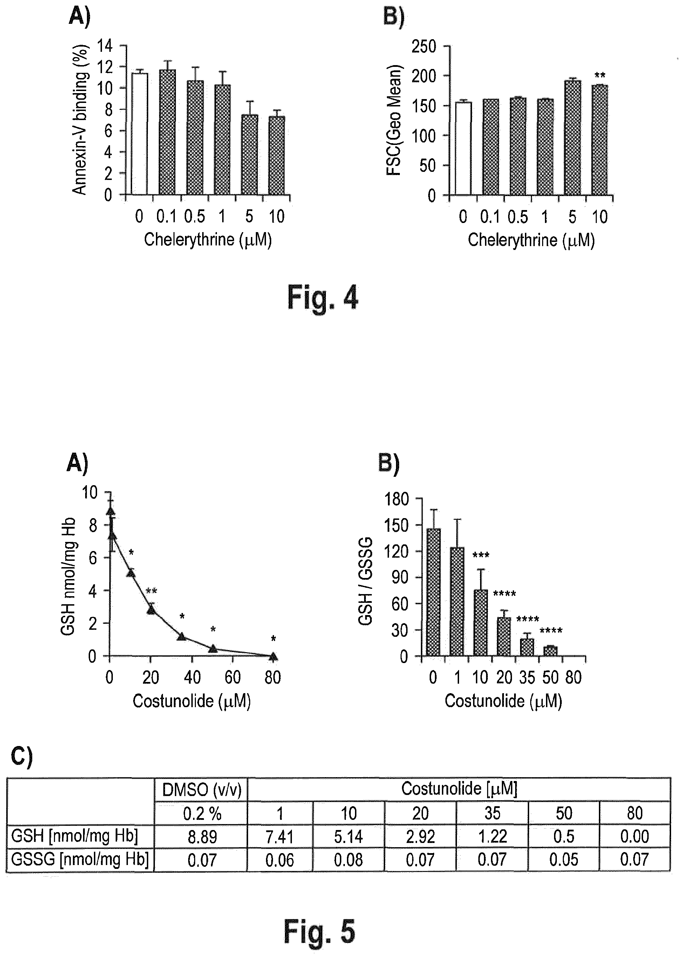

For single (Figure 5) or double treatments (Figure 7), pure RBCs (0.6% Hct) were suspended in 30 ml Ringer solution and treated with varying concentrations of costunolide (1-80 $\mu$M) or different concentrations of chelerythrine (1-10 $\mu$M) and a fixed costunolide concentration (80 $\mu$M). DMSO-treated RBCs served as negative control. After incubation, 58 $\mu$l from a 310 mM NEM stock was given to each condition, gently mixed for 1 min and then centrifuged 10 min long at 10 C and 228 x g. Supernatants were removed and the cell pellets were stored at -20 °C until analyses. GSH and GSSG were measured on the clear supernatant obtained by treatment of 0.1 ml RBCs with 0.12 ml 15% (w/v) trichloroacetic acid. For GSH analysis one aliquot (0.05 ml) of supernatant was loaded onto HPLC and the GS-NEM conjugate was revealed by a diode-array detector at 265 nm wavelength (Giustarini D. etal. 2011; Anal. Biochem. 415: 81-83, "Detection of glutathione in whole blood after stabilization with N-ethylmaleimide"). GSSG was measured at the spectrophotometer by the GSH recycling method with slight modifications (Giustarini D. et al. 2013; Nat. Protoc. 8: 1660-1669, "Analysis of GSH and GSSG after derivatization with N-ethylmaleimide"). One aliquot of RBCs (10 ml) was hemolyzed by a 1:200 dilution with $H_2O$ for haemoglobin determination (Di Iorio E.E. 1981; Methods Enzymol. 76: 57-72 "Preparation of derivatives of ferrous and ferric hemoglobin"). The HPLC analyses were carried out by an Agilent series 1100 instrument. The spectrophotometric analyses were performed by a Jasco V-530 instrument.

*Determination of G6PDH activity*

Pure RBCs (0.6% Hct) were incubated in 20 ml Ringer solution and treated with DMSO or different concentrations of costunolide. 24 hrs later, cell suspensions were centrifuged at 10 °C, 228 x g. Supernatants were removed and the cell pellets were stored at -80°C until analyses. G6PDH activity was measured in lysates according to methods recommended by the International Committee for Standardization in Hematology (Di Iorio E.E. 1981; *I.c.*). The activity was expressed in units per grams of hemoglobin (U/g Hb) (Ghashghaeinia M. et al. 2012; *I.c.*). RBC pellet was diluted 1:20 with distilled water. After 10 min (for GR 20 min) incubation at 37°C, the reactions were started by adding substrate or hemolysate and mixed properly. Then, 200 $\mu$L of each reaction mixture was transferred into a 96-well plate and changes in absorbance were recorded at 340 nm at 1 min intervals for 20 min at 37°C (Infinite 200 Nanoquant, Tecan). All assays were run in triplicates and specific enzyme activity was calculated using the Lambert-Beer law.

$$\text{Activity} = (\Delta A \times V/(\varepsilon \times l \times t)) \times f.$$

*Compositions of the assay mixtures*

*6PGA assay*

0.1 M Tris-HCI/0.5 mM EDTA, pH=8.0; 0.01 M MgCL2; 0.2 mM $NADP^+$; 20 $\mu$L of 1:20 hemolysate; 10 min incubation at 37°C; start by addition of 0.6 mM 6PGA; 20 min measurement, blank without 6PGA.

*G6PDH assay*

0.1 M Tris-HCI/0.5 mM EDTA, pH=8.0; 0.01 M MgCl2; 0.2 mM $NADP^+$; 20 $\mu$L of 1:20 hemolysate; 10 min incubation at 37°C; start by addition of 0.6 mM 6PGA + 0.6 mM G6P

*Statistical analysis*

Data are presented as the mean values $\pm$ SEM of at least 3 independent experiments with different blood samples. A total of 19 different blood samples were used in this study. One-way ANOVA with Dunnet's post test was used for statistical comparisons of treated samples with controls. Differences of the means were considered to be statistically significant when the calculated p value was less than 0.05 (*$P < 0.05$, **$P < 0.01$, ***$P < 0.001$, ****$P < 0.0001$).

2. Results

Chelerythrine is a potent, selective and cell-permeable inhibitor of the protein kinase C alpha (PKC-$\alpha$); Herbert J.M. et al., 1990, *l.c.,* and Hu B. et al., 2017, *l.c.* Human erythrocytes possess four isoforms of PKCs: alpha, zeta, mu and iota, of which only the subtype PKC-$\alpha$ translocates to the plasma membrane in order to perform biological (physiological) activities there (Govekar R.B. and Zingde S.M. 2001; Ann. Hematol. 80: 531-534, "Protein kinase C isoforms in human erythrocytes"); i.e. induction of eryptosis.

Therefore, the addition of the pharmacological substance chelerythrine (a natural product of plant origin) to blood bags (erythrocyte concentrates) is an ideal way and an effective measure to curb the increasing intensity of eryptosis during the storage of blood preserves. Thus, the blood recipients receive better, higher quality and more functional erythrocytes, thus saving their lives and relieving their immune system.

The sketch depicted in Fig. 1 is intended to illustrate the problem of weeks of blood storage and the associated negative consequences as well as the advantages of adding chelerythrine to blood bags.

In order to prove the correctness of this assumption as well as the necessity and effectiveness of adding chelerythrine to blood bags (erythrocyte concentrates), human erythrocytes were submitted to a stress test.

For this purpose, human erythrocytes were treated with the most various physiological concentrations of the natural product costunolide (a sesquiterpene) and after 24 hours its influence on eryptosis, cell shrinkage and hemolysis were evaluated. Increasing concentrations of costunolide were associated with increased rates of eryptosis and cell shrinkage (Fig. 2).

Increasing concentrations of costunolide partially simulated the increasing concentrations of stress factors that occur during the weeks of storage of human erythrocytes in blood bags, which in turn trigger the eryptosis machinery.

The second step was to examine whether chelerythrine, a natural benzophenanthridine alkaloid, can inhibit costunolide-induced eryptosis and cell shrinkage. To achieve this aim, human erythrocytes were first treated for two hours with various concentrations of chelerythrine (1 to 10 $\mu$M) and then the highest physiological concentration of costunolide (80 $\mu$M) was added. In fact, chelerythrine was able to inhibit costunolide-induced eryptosis and cell shrinkage (Fig. 3). The incubation time for chelerythrine-treated erythrocytes lasted 26 h and 24h for costunolide-treated erythrocytes. DMSO-treated erythrocytes served as a control group.

It is to be noted that the addition of chelerythrine even reduces the basal eryptosis rate: DMSO (1.69%); chelerythrine (1.1%) (Fig. 3A). As a result, chelerythrine will inhibit both basal and storage-related activation of the eryptosis machinery in blood bags (erythrocyte concentrates).

The most important aspect of this invention concerns its applicability in transfusion medicine. Chelerythrine is added to blood bags in a standardized procedure. This corresponds to the pre-incubation of human erythrocytes with chelerythrine in the experimental setup.

Obviously, chelerythrine (0.5 to 10 $\mu$M) is able to downregulate any kind of stressor-induced eryptosis and cell shrinkage (Fig. 4). This experiment served as proof of principle.

The next step was to investigate if costunolide-induced eryptosis is caused by impairment of the redox balance of human erythrocytes. For this, the influence of costunolide on glutathione (GSH) levels as well as on the activity of the pro-survival enzyme glucose-6-phosphate dehydrogenase (G6PDH) was studied. Costunolide was indeed able to deplete both the reduced (GSH) and the oxidized (GSSG) form of glutathione in a concentration-dependent manner (Fig. 5) and to inhibit G6PDH activity completely (Fig. 6).

Moreover, it was investigated if chelerythrine can affect the costunolide-induced GSH depletion. This question could be answered with a clear "no" (Fig. 7). This demonstrates that in the experimental setup chelerythrine acts downstream of the glutathione cascade and has no influence on GSH synthesis machinery. In other words: chelerythrine specifically inhibits the activity of the enzyme PKC-$\alpha$ in human erythrocytes.

The complete results of this work are shown in Fig. 8.

3. Conclusions

The inventor has surprisingly realized that chelerythrine, a natural product of plant origin, or derivatives thereof can be used *in vitro* as a beneficial active ingredient of an erythrocytes-containing composition, such as a blood conserve. The addition of the PKC inhibitor chelerythrine to an erythrocytes-containing composition can/will extend the storage durability of blood bags, support the vitality and functional efficiency of human erythrocytes and strongly inhibit the storage-related increase of eryptosis machinery and the intensification of hemolytic processes. As a result, the recipients of the erythrocyte concentrates receive better, higher quality and more functional erythrocytes, saving their lives and relieving their immune system.

The advantages of adding the chelerythrine to erythrocytes-containing or blood conserves (erythrocyte concentrates), which may be stored in a standardized erythrocyte-specific preservation solution (the so-called stabilizing additive solution), as discovered by the inventor, can be summarized as follows:

a) Achieving better vitality and functionality of human erythrocytes;

b) Significant inhibition of storage-related eryptosis (erythrocyte death);

c) Significant inhibition of calcium-dependent activation of cation channels;

d) Significant inhibition of the storage-related shrinkage of human erythrocytes;

e) Increasing the durability of human erythrocytes;

f) Significant reduction of storage-related hemolytic processes in human erythrocytes;

g) Significant relief of the immune system of the blood recipient.

**Claims**

1. Use of chelerythrine or a derivative thereof *in vitro* as an active ingredient of an erythrocytes-containing composition.

2. Use according to claim 1, **characterized in that** said erythrocytes-containing composition is a blood conserve.

3. Use according to claim 1 or 2, **characterized in that** said composition and/or blood conserve is configured for a non-cryopreserved storage.

4. Use according to any of the preceding claims, **characterized in that** said chelerythrine or derivative thereof supports the viability and/or functional efficiency of said erythrocytes.

5. Use according to any of the preceding claims, **characterized in that** said chelerythrine or derivative thereof inhibits eryptosis and/or hemolysis.

6. Use according to any of the preceding claims, **characterized in that** said chelerythrine or derivative thereof increases the shelf-life of said composition and/or blood conserve.

7. Use according to any of the preceding claims, **characterized in that** said chelerythrine or derivative thereof is contained in the composition and/or blood conserve in a concentration of approx. $\geq 0.5$ $\mu$M, preferably of approx. $\geq 1$ $\mu$M, further preferably of approx. $\geq 5$ $\mu$M, and highly preferably of approx. $\geq 10$ $\mu$M.

8. Erythrocytes-containing composition comprising chelerythrine or a derivative thereof as an active ingredient.

9. Erythrocytes-containing composition of claim 8, which is a blood conserve.

10. Erythrocytes-containing composition of claim 8 or 9, **characterized in that** said composition and/or blood conserve is configured for non-cryopreserved storage.

11. Erythrocytes-containing composition of any of the preceding claims for use in the treatment and/or prophylaxis of blood loss in a human and/or animal being.

12. Erythrocytes-containing composition of claim 11, **characterized in that** said use is for the treatment and/or prophylaxis of acute blood loss.

13. Erythrocytes-containing composition of any of the preceding claims for use in the treatment and/or prophylaxis of a blood-formation disorder.

14. Method of treatment of a human or animal being suffering from and/or threatened by blood loss, preferably acute blood loss, comprising the administration of the erythrocytes-containing composition of any of claims 8-13 to said being.

15. Method of treatment of a human or animal being suffering from and/or threatened by a blood-formation disorder, comprising the administration of the erythrocytes-containing composition of any of claims 8-13 to said being.

Chelerythrine
⊥

GSH depletion / or stress factors / senescence → PKC-α activation → erythrocytes death (eryptosis)

## Fig. 1

**A)** ☐ 24 h

DMSO      80 μM Costunolide

1.75      61.26

Annexin V

**B)** ☐ 24 h

**C)** ☐ 24 h

**D)** ☐ 24 h

DMSO      80 μM Costunolide

GeoMean: 270      GeoMean: 185

FSC

**E)** ☐ 24 h

## Fig. 2

Fig. 3

## A)

## B)

## Fig. 4

## A)

## B)

## C)

| | DMSO (v/v) | Costunolide [µM] | | | | | |
|---|---|---|---|---|---|---|---|
| | 0.2 % | 1 | 10 | 20 | 35 | 50 | 80 |
| GSH [nmol/mg Hb] | 8.89 | 7.41 | 5.14 | 2.92 | 1.22 | 0.5 | 0.00 |
| GSSG [nmol/mg Hb] | 0.07 | 0.06 | 0.08 | 0.07 | 0.07 | 0.05 | 0.07 |

## Fig. 5

A)

B)

**Fig. 6**

A)

B)

**Fig. 7**

**Fig. 8**

Europäisches
Patentamt

European
Patent Office

Office européen
des brevets

# EUROPEAN SEARCH REPORT

Application Number

EP 20 15 5897

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 99/60849 A1 (LIFECELL CORP [US]; LIVESEY STEPHEN ANTHONY [AU] ET AL.) 2 December 1999 (1999-12-02) | 1-15 | INV. A61K9/00 A61K35/14 |
| Y | * the whole document * * claims 1-28 * | 1-15 | |
| X | US 4 356 172 A (NAKAO MAKOTO ET AL) 26 October 1982 (1982-10-26) | 1-15 | |
| Y | * the whole document * * claims 1-4 * | 1-15 | |
| Y | NATALIA IVANOVNA AGALAKOVA ET AL: "Effects of phorbol 12-myristate 13-acetate on potassium transport in the red blood cells of frog Rana temporaria", JOURNAL OF COMPARATIVE PHYSIOLOGY B ; BIOCHEMICAL, SYSTEMIC, ANDENVIRONMENTAL PHYSIOLOGY, SPRINGER, BERLIN, DE, vol. 179, no. 4, 28 December 2008 (2008-12-28), pages 443-450, XP019711555, ISSN: 1432-136X * the whole document * | 1-15 | |
| Y | TANG FUZHOU ET AL: "Ankyrin exposure induced by activated protein kinase C plays a potential role in erythrophagocytosis", BIOCHIMICA ET BIOPHYSICA ACTA (BBA) - GENERAL SUBJECTS, ELSEVIER, AMSTERDAM, NL, vol. 1860, no. 1, 22 October 2015 (2015-10-22), pages 120-128, XP029325046, ISSN: 0304-4165, DOI: 10.1016/J.BBAGEN.2015.10.017 * the whole document * | 1-15 | TECHNICAL FIELDS SEARCHED (IPC) A61K |

-/--

The present search report has been drawn up for all claims

| Place of search | Date of completion of the search | Examiner |
|---|---|---|
| Munich | 8 July 2020 | Felder, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 20 15 5897

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GENNADII PETROVICH GUSEV ET AL: "Regulation of K-Cl cotransport in erythrocytes of frog Rana temporaria by commonly used protein kinase and protein phosphatase inhibitors", JOURNAL OF COMPARATIVE PHYSIOLOGY B ; BIOCHEMICAL, SYSTEMIC, ANDENVIRONMENTAL PHYSIOLOGY, SPRINGER, BERLIN, DE, vol. 180, no. 3, 20 November 2009 (2009-11-20), pages 385-391, XP019780142, ISSN: 1432-136X * the whole document * | 1-15 | |
| Y | TEIXEIRA PEDRO ET AL: "Acetylcholinesterase Conformational States Influence Nitric Oxide Mobilization in the Erythrocyte", JOURNAL OF MEMBRANE BIOLOGY, SPRINGER NEW YORK LLC, US, vol. 248, no. 2, 5 February 2015 (2015-02-05), pages 349-354, XP035443942, ISSN: 0022-2631, DOI: 10.1007/S00232-015-9776-Y [retrieved on 2015-02-05] * the whole document * | 1-15 | TECHNICAL FIELDS SEARCHED (IPC) |

The present search report has been drawn up for all claims

| Place of search | Date of completion of the search | Examiner |
|---|---|---|
| Munich | 8 July 2020 | Felder, Christian |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 20 15 5897

This annex lists the patent family members relating to the patent documents cited in the above-mentioned European search report.
The members are as contained in the European Patent Office EDP file on
The European Patent Office is in no way liable for these particulars which are merely given for the purpose of information.

08-07-2020

| Patent document cited in search report | | Publication date | Patent family member(s) | | Publication date |
|---|---|---|---|---|---|
| WO 9960849 | A1 | 02-12-1999 | AT | 316757 T | 15-02-2006 |
| | | | AU | 758703 B2 | 27-03-2003 |
| | | | CA | 2332986 A1 | 02-12-1999 |
| | | | DE | 69929691 T2 | 07-09-2006 |
| | | | EP | 1082006 A1 | 14-03-2001 |
| | | | ES | 2257050 T3 | 16-07-2006 |
| | | | JP | 2002516254 A | 04-06-2002 |
| | | | US | 2006127375 A1 | 15-06-2006 |
| | | | US | 2012141974 A1 | 07-06-2012 |
| | | | WO | 9960849 A1 | 02-12-1999 |
| US 4356172 | A | 26-10-1982 | JP | S56139419 A | 30-10-1981 |
| | | | US | 4356172 A | 26-10-1982 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9960849 A

- US 5137912 A

### Non-patent literature cited in the description

- **GHASHGHAEINIA M. et al.** The impact of erythrocyte age on eryptosis. *Br. J. Haematol.,* 2012, vol. 157, 606-614
- **GHASHGHAEINIA M. et al.** Age sensitivity of NFκB abundance and programmed cell death in erythrocytes induced by NFκB inhibitors. *Cell. Physiol. Biochem.,* 2013, vol. 32, 801-813
- **GHASHGHAEINIA M. et al.** Trifluoperazine-Induced Suicidal Erythrocyte Death and S-Nitrosylation Inhibition, Reversed by the Nitric Oxide Donor Sodium Nitroprusside. *Cell. Physiol. Biochem.,* 2017, vol. 42, 1985-1998
- **HERBERT J.M. et al.** Chelerythrine is a potent and specific inhibitor of protein kinase C. *Biochem Biophys Res Commun.,* 1990, vol. 172, 993-999
- **HU B. et al.** Chelerythrine Attenuates Renal Ischemia/Reperfusion-induced Myocardial Injury by Activating CSE/H2S via PKC/NF-κB Pathway in Diabetic Rats. *Kidney Blood Press Res.,* 2017, vol. 42, 379-388

- *CHEMICAL ABSTRACTS,* 34316-15-9
- **LANG P.A. et al.** Stimulation of erythrocyte ceramide formation by platelet-activating factor. *J. Cell. Sci.,* 2005, vol. 118, 1233-1243
- **GIUSTARINI D. et al.** Detection of glutathione in whole blood after stabilization with N-ethylmaleimide. *Anal. Biochem.,* 2011, vol. 415, 81-83
- **GIUSTARINI D. et al.** Analysis of GSH and GSSG after derivatization with N-ethylmaleimide. *Nat. Protoc.,* 2013, vol. 8, 1660-1669
- **DI IORIO E.E.** Preparation of derivatives of ferrous and ferric hemoglobin. *Methods Enzymol.,* 1981, vol. 76, 57-72
- **GOVEKAR R.B. ; ZINGDE S.M.** Protein kinase C isoforms in human erythrocytes. *Ann. Hematol.,* 2001, vol. 80, 531-534